**Europäisches Patentamt**

**European Patent Office**

**Office européen des brevets**

(11) Publication number : **0 520 834 A2**

(12) # EUROPEAN PATENT APPLICATION

(21) Application number : **92305970.3**

(51) Int. Cl.⁵ : **A61K 37/02, A61K 39/395**

(22) Date of filing : **29.06.92**

(30) Priority : **28.06.91 US 720591**

(43) Date of publication of application :
**30.12.92 Bulletin 92/53**

(84) Designated Contracting States :
**BE CH DE ES FR GB IT LI NL SE**

(71) Applicant : **INTERMUNE LIFE SCIENCES, INC.**
**108 Woodbine Downs, Unit 2**
**Etobicoke, Ontario (CA)**

(72) Inventor : **Drizen, Alan Suite 1201**
**100 Canyon Avenue**
**Downsview, Ontario (CA)**
Inventor : **Bolton, Anthony E.**
**12 Thornsett Road**
**Sheffield, S7 INB (GB)**

(74) Representative : **Jump, Timothy John Simon et al**
**Venner Shipley & Co. 20 Little Britain**
**London EC1A 7DH (GB)**

(54) **Pharmaceutical composition for treating an immune system disorder comprising PP14 or antibodies against PP14.**

(57)   A method for treating an immune system disorder in a human by administering to the human a therapeutically effective amount of an active substance selected from the group consisting of PP14, derivatives of PP14, muteins of PP14, fragments of PP14, and subunits of PP14, to alleviate the immune system disorder.

EP 0 520 834 A2

The present invention relates to inhibitors of immune cell proliferation and function. More particularly, the present invention is directed to the use of PP14 as an inhibitor of immune cell proliferation and function.

The human immune system functions to protect the organism from infection and from foreign antigens by cellular and humoral mechanisms. Interleukin 1 (IL-1) is a peptide cytokine secreted by a variety of cell types, including accessory cells of the immune system and antigen presenting cells. It now appears established that IL-1 is a central mediator of inflammatory reactions and is important in the pathogenesis of chronic inflammatory diseases, of which rheumatoid arthritis (RA) is one example. Thus an inhibitor of IL-1, therefore, may be of importance in the treatment of this disease and other inflammatory diseases. One potential such inhibitor has been identified in placental protein 14 (PP14), which is a natural product present at elevated levels in the peripheral circulation early in pregnancy, peaking around week 9-10. A detailed discussion of the immune system, the role played in that system by IL-1, and the possible inhibitory action of PP14 is included in EP-A-0378418. This document also discloses the possibility of using PP14, derivatives, fragments and subunits thereof, or monoclonal antibodies to PP14 or derivatives, fragments or subunits thereof, in the treatment of immune system, autoimmune, allergic, neoplastic and inflammatory disorders. Said antibodies are also described as being useful in methods of testing for PP14.

In accordance with a first aspect of the present invention there is provided a pharmaceutical preparation for treating an immune system disorder in a human, comprising an active substance selected from the group consisting of PP14, derivatives of PP14, fragments of PP14, and subunits of PP14, characterised in that:

said PP14 comprises a polypeptide including an amino acid sequence as shown in SEQID NO:1;

said derivatives of PP14 comprise polypeptides including amino acid sequence as shown in SEQID NO:1, or at least one sequential subset thereof, and/or active muteins of PP14 comprising a mutant of a polypeptide including an amino acid sequence as shown in SEDID NO:1, or at least one sequential subset thereof;

said fragments of PP14 comprise polypeptides including a sequential subset of the amino acid sequence as shown in SEQID NO:1;

said subunits of PP14 include one polypeptide having substantially all the amino acid sequence as shown in SEQID NO:1; and

said substance is effective in alleviating said immune system disorder.

In a second aspect, the present invention provides a pharmaceutical preparation for treating an immune system disorder in a human, comprising an active substance, characterised in that said substance is a derivative of PP14 which, preferably, comprises a polypeptide including an amino acid sequence as shown in SEQID NO:1, or at least one sequential subset thereof, and/or an active mutein of PP14 comprising a mutant of a polypeptide including an amino acid sequence as shown in SEQID NO:1, or at least one sequential subset thereof, augmented by at least one additional molecule selected from the group consisting of glucose moieties, lipids, phosphate groups, acetyl groups, hydroxyl groups, saccharides, methyl groups, propyl groups, amino acids, and polymeric molecules.

In a preferred embodiment of either aspect of the invention, at least one amino acid residue in the polypeptide has been modified by oxidation or reduction. Preferably, the PP14 comprises a dimer of two non-covalently linked protein subunits and, morre preferably, at least one of said subunits is employed as the active substance in the inventive pharmaceutical preparation. Most preferably, the subunit has an amino acid sequence as shown in SEQID NO:1.

In a further embodiment, the PP14 employed in pharmaceutical preparations of either aspect of the invention, comprises two covalently linked protein. subunits, each of wich subunits has an amino acid sequence as shown in SEQID NO:1. Preferably, where a fragment of PP14 comprises the active substance in a pharmaceutical preparation, in accordance with either aspect of the present invention, said fragment has an amino acid sequence comprising residues 63-160, or residues 80-105, of the sequence shown in SEQID NO:1.

Pharmaceutical preparations in accordance with the invention may be used in the treatment of various immune system disorders, including allergic conditions, autoimmune conditions and inflammatory conditions; preferably, the compositions are used to treat disorders including arthritis, rheumatoid arthritis, asthma, graft-versus-host disease, organ rejection, systemic lupus erythematosis, atopic allergy, inflammatory bowel disease, multiple sclerosis and, allergic dermatitis.

The inventive compositions are also useful in treating autoimmune conditions manifested by infertility, lymphoproliferative disorders, including malignant non-Hodgkin's lymphoma, Hodgkin's disease, and malignant histiocytosis, and neoplatic disorders, such as leukemia. The preparations can also be useful in treating disorders resulting from the presence in a human of the virus which causes acquired immunodeficiency syndrome.

In a third aspect, the present invention provides a pharmaceutical preparation for treating an immune system disorder in a human, characterised by comprising monoclonal antibodies directed against an active substance as employed in the pharmaceutical preparations of the first and second aspects of the invention, said monoclonal antibodies being effective in alleviating the immune system disorder.

2

The immune system disorders treatable using a pharmaceutical preparation in accordance with the third aspect of the invention, include those caused by the presence in a human of the virus which causes acquired immunodeficiency syndrome, in addition to many of the other aforementioned conditions.

Pharmaceutical preparations, in accordance with any aspect of the invention, preferably, are in a form suitable for administration by means selected from the group consisting of intravenous injection, intramuscular injection, oral administration, rectal administration and, inhalation. Preferably, the active substance in the preparation of the first and second aspects of the invention and from which the antibodies of the third aspect are prepared, is obtained from a source selected from the group consisting of mammalian placenta, mammalian blood, amniotic fluid, seminal plasma, cells in tissue culture, decidual cells, decidual organs, endometrial cells, endometrial organs, and sources containing eukaryotic cells or prokaryotic cells engineered to express PP14, muteins of PP14, fragments of PP14 or subunits of PP14.

Preferred features and embodiments of the invention are described and defined in the sub-claims appended hereto.

The discoveries of the present invention may also be applied to test inflammatory and autoimmune diseases and, in another embodiment of the invention, a hybridoma cell line producing monoclonal antibodies, for use in accordance with the third aspect of the invention, is provided.

In a further embodiment, the present invention can involve a method for the detection and quantification of PP14, the method which comprises:

(a) contacting a sample suspected of containing PP14 with a composition comprising antibodies, suitable for use in accordance with the third aspect of the present invention; and

(b) subjecting the sample to an assay to detect the presence and amount of any antibody-antigen reaction therein.

The assay may be selected from the group consisting of radioimmunoassay, ELISA assays, and immunoblotting assays.

The present invention further includes a method for purifying PP14 from a substance containing PP14, the method which comprises contacting the substance with nonoclonal antibodies directed against the PP14, whereby an immunoprecipitation reaction, or antigen: antibody interaction results.

PP14 suitable for purposes of the present invention binds to the monoclonal antibody used in accordance with the present invention. The activities observed from in vitro test sytems correlate with the PP14 content of tissue extracts and other preparations, as measured in a radioimmunoassay for PP14 that utilises a polyclonal antibody. This radioimmunoassay is described in the publication by Anthony E. BOLTON et al., entitled "The radioimmunoassay of Human Placental Protein 14 (PP14), "Clinica Chimica Acta , 135 (1983) 283-291. Useful forms of PP14 for purposes of the present invention include natural and recombinant forms of PP14 itself, as well as derivatives, muteins, fragments, and subunits of PP14, provided that the desired therapeutic activity of the substance is maintained.

PP14 is a glycoprotein comprising about 17.5% carbohydrate content. The details of this carbohydrate content are not presently known; however, PP14 binds strongly to the lectin concanavalin-A, which is known to have an affinity for terminal alpha-D-mannosyl and alpha-D-glucosyl residues, as well as to wheat germ agglutinin, which has an affinity for N-acetyl-beta-D-glucosaminyl residues. The presence of the latter is further evidenced by the reduction of the interaction of PP14 with specific antibodies caused by treatment of PP14 with the enzyme beta-N-acetyl glucosaminidase, which removes these residues.

The nucleotide and amino acid sequence of PP14 cDNA, as deduced by Julkunen et al., is shown in the accompanying Sequence Description (SEQ ID NO:1). The entire disclosure of Julkunen et al., "Complete Amino Acid Sequence of Human Placental Protein 14: A Progesterone-Regulated Uterine Protein Homologous to β-lactoglobulins," Proc. Natl. Acad. Sci. USA, Vol. 85, pp. 8845-8849, December 1988, is incorporated herein by reference.

It will be understood that the precise chemical structure of PP14 depends upon a number of factors. For example, since ionizable amino and carboxyl groups are present in the molecule, a particular protein may be obtained as an acidic or basic salt, or in neutral form. All forms of PP14 which retain their therapeutic activity for purposes of the instant invention are intended to be within the scope of the definition of "PP14".

It should be noted that the N-terminal amino acid sequence of PP14 shows substantial sequence homology with certain animal β-lactoglobulins, but the biological activities of these proteins are not well understood. Furthermore, there is some sequence homology between PP14 and human serum retinol binding protein.

The term "recombinant" used herein refers to PP14 produced by recombinant DNA techniques wherein the gene coding for the PP14 is cloned by known recombinant DNA technology. For example, the human gene for PP14 may be inserted into a suitable DNA vector, such as a bacterial plasmid, and the plasmid used to transform a suitable host. The gene is then expressed in the host to produce the recombinant protein. The transformed host may be prokaryotic or eukaryotic, including mammalian, yeast, Aspergillus and insect cells. One preferred embodiment employs bacterial cells as the host.

Therapeutically useful derivatives of PP14 may be prepared by augmenting the primary amino acid sequence of the protein PP14 with at least one additional molecule selected from the group consisting of glucose moieties, lipids, phosphate groups, acetyl groups, hydroxyl groups, saccharides, methyl groups, propyl groups, amino acids, and polymeric molecules. Augmentation may be accomplished through post-translational processing systems of the producing host, or it may be carried out in vitro. Both techniques are well-known in the art.

Referring to the Sequence Description of PP14, it should be noted that the peptide includes three potential glycosylation sites at amino acid residues 28-30, 63-65, and 85-87. Glycosylation is a process of forming a protein derivative, wherein a portion of the protein's amino acid sequence is augmented by a sugar moiety. It will therefore be understood that therapeutically useful derivatives of PP14 may be prepared by addition of one or more sugar residues to the protein, or alternatively by removal of some or all of the sugar residues from the sites of glycosylation on the PP14 molecule.

Other therapeutically useful derivatives of PP14 may be formed by modifying at least one amino acid residue of PP14 by oxidation, reduction, or other derivatization processes known in the art.

Muteins of PP14 which do not destroy the activity of the protein may be used as the active treating substance of the instant invention. Muteins are prepared by modification of the primary structure of the protein itself, by deletion, addition, or alteration of the amino acids incorporated into the sequence during translation. For example, at least one cysteine residue of PP14 may be replaced with a conservative amino acid, in order to eliminate sites of undesirable intramolecular disulfide bond formation. Crosslinking is undesirable if it changes the conformation of PP14 so as to render the protein essentially inactive for purposes of treating an immune system disorder. Also, it may be desirable to replace a methionine which is not essential to bioactivity with a conservative amino acid. As referred to herein, a conservative amino acid alteration is defined as one which does not significantly adversely affect biological activity and involves substitutions of the amino acid. The conservative amino acid that may be substituted for cysteine and methionine include at least: serine, alanine, glycine, valine, threonine, leucine, isoleucine, and tyrosine. More preferably they include serine and alanine. Most preferably, cysteine may be replaced with serine and methionine replaced with alanine.

Placental protein 14 is believed to exist in nature as a dimer of two identical, non-covalently linked protein subunits. Accordingly, since each subunit is believed to have the amino acid sequence shown in SEQ ID NO:1, a subunit of PP14 could be used as the therapeutic active substance for treating human immune system disorders according to the instant invention. The invention also encompasses use of subunits of PP14 that are covalently linked, either naturally or by artificial techniques known in the art.

In addition, it is contemplated that fragments of PP14 would be useful for treating human immune system disorders, provided that such fragments retained their therapeutic activity. Referring to SEQ ID NO:1, the protein fragment defined by amino acid residues 63 through 160 is believed to be a therapeutically active fragment of PP14 for purposes of the invention. This fragment includes four cysteine amino acids, which are believed to have biological activity. The fragment defined by amino acid residues 80 through 105 is also believed to be therapeutically active for purposes of the invention. The fragment defined by residues 80-105 is believed to be therapeutically active because: it is a linear sequence not involving disulfide bridges; the fragment contains a glycosylation site (at residues 85-87); the fragment has two tyrosine residues which, because of their phenyl side chains, may be involved in receptor interaction; and the double lysine sequence at residues 80-81 have double amino acid group activity, suggesting potential receptor activity.

The above-described forms of PP14 are used in an effective therapeutic amount, which will vary depending on the particular immune system disorder being treated, the method of administration, the form of PP14 utilized, and other factors understood by those having ordinary skill in the art. For example, treatment of arthritis may require direct injection of PP14 into the joints in order to achieve a suitable therapeutic dose, whereas a topically administered preparation for the treatment of psoriasis may require a different dosage of PP14. In general, one of ordinary skill in the art will be able to arrive at therapeutically effective dosages of PP14 for treatment of the various immune system disorders contemplated by the invention, based on the range of concentrations of PP14 which has been discovered to provide suitable in vitro activity, namely from about 0.1 to about 10 micromole/liter.

The activation of lymphocytes, resulting in cell proliferation, is a complex response mediated by a number of peptide messengers, the cytokines. At a simplified level, T-lymphocytes are activated by a sequential process. First, there is a requirement for the cytokine Interleukin-1 (IL-1) secreted by accessory cells (e.g., cells of the monocyte/macrophage lineage). In the presence of IL-1 the cytokine Interleukin-2 (IL-2) increases the expression of its own receptors, making the cell more responsive to IL-2 -- a positive feedback cycle. IL-2 also stimulates T-cell division (lymphoproliferation). Thus, the proliferation of T-cells requires the presence of both IL-1 and IL-2.

One approach to investigating the mode of action of lymphoproliferation with an inhibitor such as PP14 is to add an excess of cytokine along with the inhibitor and determine whether the suppression is reversed. One source of a crude mixture of cytokines is the supernatant taken from cultured activated lymphocytes. Such supernatants were demonstrated to reverse the suppressive action of PP14, indicating a mode of action relating to the secretion/activity of cytokines. The addition of recombinant IL-1 at a single dose significantly reduced the suppressive action of PP14 on lymphoproliferation, as shown in the following Table:

## Table 1

This table shows the effect of the addition of 5U/ml of recombinant IL-1 on the suppression of tritiated thymidine uptake by stimulated lymphocytes.

| Decidual sample no. | PP14 (ng/ml) | %suppression of 3H-Tdr | |
|---|---|---|---|
| | | +IL-1 | -IL-1 |
| DE A | 5.0 | 25 | 30 |
| DE B | 4.8 | 12 | 62 |
| DE C | 4.0 | 25 | 46 |
| DE D | 8.0 | 33 | 48 |
| DE E | 2.0 | 30 | 41 |

Mean (+/-S.D.)

These data indicate that PP14 may be operating via an IL-1-mediated mechanism.

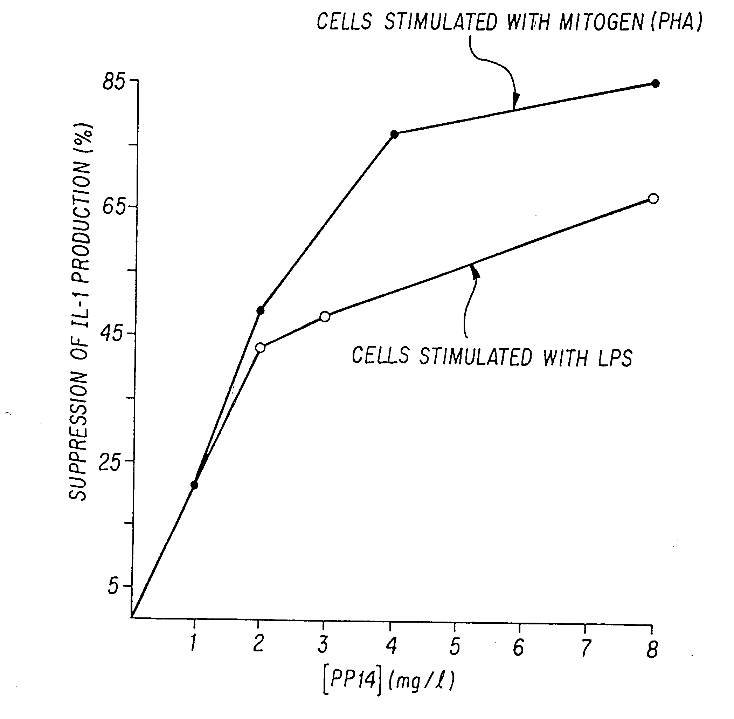

To investigate this possibility further, peripheral blood mononuclear cells (a mixture primarily of T-cells and monocytes) were activated using the mitogen PHA in the presence and absence of inhibitory amounts of PP14. The amount of IL-1 released into the supernatants of the cultured cells was measured after different times of culture. The results from the two experiments carried out are shown in the attached Figure, and the data are given in Table 2 below. It can be seen that PP14 significantly inhibited the release of IL-1 into the supernatant by activated cells. These data on IL-1 strongly suggest that PP14 is acting at the IL-1 level of T-cell activation by inhibiting its synthesis/release.

## Table 2

This Table shows the effect of PP14 in decidual extracts on the release into cell culture supernatants of IL-1 by stimulated peripheral blood lymphocytes.

| Experiment 1 | TIME (hours) | | | |
|---|---|---|---|---|
| | 22.5 | 41 | 65 | 89 |
| Unstimulated | 0.2 | 0.2 | 0.1 | 0.1 |
| Immunoabsorbed extract | 1.583 | 1.266 | 1.232 | 1.196 |
| Unabsorbed extract | 0.406 | 0.216 | 0.212 | 0.2 |
| % Suppression | 75% | 83% | 91% | 83% |

| Experiment 2 | TIME (hours) | | | |
|---|---|---|---|---|
| | 18 | 42 | 66 | 80 |
| Unstimulated | 0.6 | 0.2 | 0.4 | 0.3 |
| Immunoabsorbed extract | 1.789 | 2.554 | 2.709 | 2.807 |
| Unabsorbed extract | 0.7 | 1.162 | 1.630 | 1.967 |
| % Suppression | 61% | 55% | 40% | 30% |

NOTES

Unstimulated -- spontaneous release of IL-1 from unstimulated lymphocytes.

Immunoabsorbed extract -- IL-1 release from stimulated cells in the presence of a crude decidual extract from which

PP14 had been specifically removed by monoclonal antibody immunoabsorption.

Unabsorbed extract -- IL-1 release from stimulated lymphocytes in the presence of a crude decidual extract containing 8.0 ug/ml of PP14.

Percent suppression -- the suppression of IL-1 released into the cell culture supernatants by PP14 in decidual extracts, expressed as a percent of the release of IL-1 in the presence of decidual extracts from which PP14 had been removed.

Individual results for each experiment are means of triplicate determinations.

A monoclonal antibody which specifically binds to PP14 has been isolated and characterized. This monoclonal antibody is designated MAb 14/1/1, and was derived from hybridomal cell lines produced by fusion, using a polyethylene glycol method of spleen cells from mice immunized with crude extracts of deciduum with the myeloma cell line P3/NS1/1-Ag4-1, which is a standard myeloma cell line. Clones secreting anti-PP14 were selected using the radiolabelled PP14 used for the radioimmunoassay (BOLTON et al., 1983, supra). Positive cultures were cloned three times by limiting dilution and those yielding the highest titers used to induce tumors in Balb/C mice. IgG was isolated from ascitic fluid from ion exchange chromatography or affinity chromatography on protein-A.

The specificity of the antibody was examined in two ways. First, the two-site immunoradiometric assay was set up using a polyclonal extracting antibody and the monoclonal as labelled antibody. Polyclonal antibody, raised against crude decidual tissue extract, was covalently linked to Sepharose-4B by standard procedures. This was incubated in excess in the presence of standard PP14 or the potentially cross-reacting protein for two hours at room temperature. Subsequently, radioiodinated monoclonal antibody was added, incubated a further two hours, and the solid-coupled antibody washed and counted for radioactivity. This represents a conventional 2-site immunoradiometric assay. The cross-reaction of various decidual/placenta proteins was investigated in this system.

The following gave less than 0.1% cross reaction: hPL, SP1, pp5, pp12, pregnancy-associated plasma protein-A (PAPP-A), placental alkaline phosphatase, placental malic dehydrogenase, placental sphyngomyelinase, placental arylamidase, placental choline acetyltransferase, with only PP14 having any observed activity in this assay.

The other method involved investigating the binding to the monoclonal antibody of radioactively-labelled pure proteins. no significant binding of prolactin, hCG, or PAPP-A was detected. This antibody could be used in the assay, for example by a two-site immunoradiometric procedure, and purification of PP14. As previously mentioned, the details of the radioimmunoassay for PP14 are disclosed in the 1983 BOLTON et al. article, identified above.

The following examples are given to illustrate the invention, but are not deemed to be limiting thereof.

Example 1

Evidence that PP14 inhibits IL-1 release

Method 1 - the mitogenic response of human lymphocytes

Human peripheral blood lymphocytes proliferate in response to stimulation by the mitogen phytohaemagglutinin (PHA), the proliferation being measured by the incorporation of tritiated thymidine into the DNA of the dividing cells. This is a standard test of lymphocyte responsiveness. PP14 inhibits lymphocyte proliferation in response to PHA stimulation. The addition of recombinant IL-1 partially reverses the inhibition caused by PP14.

The test system used in this example was to investigate the effect of recombinant IL-1 on the mitogen-induced stimulation of PP14-inhibited peripheral blood lymphocytes. The use of PP14-inhibited cells in experiments such as this is unique methodology.

The PP14 inhibition of cells in this example was carried out as follows. Cells were treated with crude extracts of human decidual tissue in which the PP14 concentration was measured by radioimmunoassay. As a control for each treated cell preparation, a portion of the cells were treated with the same extract that had been immunoabsorbed using a monoclonal antibody to PP14, MAb/PP14/1/1, to remove the PP14 in a highly specific manner. The difference between the activity of the cells treated with these two preparations represented the effect of PP14. Thus, the effects measured were those of PP14, which specifically binds to this monoclonal antibody.

Peripheral blood mononuclear cells were isolated from whole blood obtained from healthy donors by a standard density gradient centrifugation method. After washing in a physiological medium, the cells were resuspended at an appropriate concentration of viable cells and incubated in the presence of the PP14 preparation and the mitogen at a stimulatory concentration.

The effects of IL-1, or other test compound, were assessed by inclusion at appropriate concentrations in this incubation, including also the necessary controls. The cells were incubated for 72 hours at 37°C in an atmosphere of 5% carbon dioxide and 100% humidity under sterile conditions. Six hours prior to termination of the cultures, the cells were pulsed with 1 uCi of tritiated thymidine, and on termination the cells were harvested automatically onto glass fiber filters.

The degree of lymphoproliferation was assessed by measuring the incorporation of tritiated thymidine into the harvested cells by liquid scintillation counting. Inhibition of tritiated thymidine uptake by PP14, and the effect of IL-1 were as follows:

## % inhibition of tritiated thymidine uptake

| Without IL-1 | With 5U/ml IL-1 |
|---|---|
| 45.4 +/- 11.6 | 25.0 +/- 8.0 |

These are mean results from 15 different experiments, each performed in triplicate, using 5 different decidual extracts as the source of PP14 and cells from 2 separate donors. The results are significantly different ($p < 0.0001$), indicating a significant reversal of the inhibitory effect of PP14 on lymphoproliferation by IL-1.

Example 2

The effect of PP14 on the production of IL-1 by stimulated peripheral blood mononuclear cells

Peripheral blood mononuclear cells, as isolated by density gradient centrifugation, contain not only lymphocytes (the majority of cells present) but also monocyte/macrophages, which are necessary accessory cells in the lymphoproliferative reaction described above. It is this latter cell type which synthesizes and secretes IL-1. These cells can be activated to secrete their specific products which include IL-1 by both mitogens (e.g., PHA) and lipopolysaccharide (LPS).

The test system used in this example was to investigate the production of macrophage/monocyte products released from stimulated PP14-inhibited peripheral blood mononuclear cell preparations, measuring the products by commercially available immunoassay systems. The use of PP14-inhibition of cells was carried out as in Method 1 of Example 1 above, which again is unique methodology.

Peripheral blood mononuclear cells were prepared as under Method 1. Appropriate numbers of cells were incubated in the presence of the PP14 preparation and the stimulator for various times under the conditions described above. At the termination of the culture period the cells were harvested and the supernatants assayed for IL-1 and Tumour Necrosis Factor (TNF), both known to be products of macrophage/monocyte cells. Typical results are summarized below.

Inhibition of IL-1 release by stimulated peripheral mononuclear cells by PP14:

## % inhibition of IL-1 release

| PHA stimulated cells | LPS stimulated cells |
|---|---|
| 82.5 | 67.0 |

These are mean results from 3 experiments.

This inhibition of IL-1 release from stimulated (using either PHA or LPS) mononuclear cells by PP14 is dose dependant, as shown in the Figure, wherein ● represents cells stimulated with mitogen (PHA) and o represents cells stimulated with LPS.

Example 3

Comparison of IL-1 and TNF production by PP14-inhibited stimulated peripheral blood mononuclear cells: Cells stimulated with mitogen:

|  | IL-1 produced | TNF produced |
|---|---|---|
| stimulated control | 2.60 ng/ml | 1043 pg/ml |
| +PP14 | 0.46 ng/ml | 1474 pg/ml |
| (4ug/1) | | |

The invention being thus described, it will be obvious that the same may be varied in many ways. Such variations are not to be regarded as a departure from the spirit and scope of the invention and all such modifications are intended to be included within the scope of the claims.

## Claims

1. A pharmaceutical preparation for treating an immune system disorder in a human comprising an active substance selected from the group consisting of PP14, derivatives of PP14, fragments of PP14, and subunits of PP14, characterised in that:

   said PP14 comprises a polypeptide including an amino acid sequence as shown in SEQID NO:1;

   said derivatives of PP14 comprise polypeptides including the amino acid sequence shown in SEQID NO:1, or at least one sequential subset thereof, and/or active muteins of PP14 comprising a mutant of a polypeptide including an amino acid sequence as shown in SEQID NO:1, or at least one sequential subset thereof;

   said fragments of PP14 comprise polypeptides including a sequential subset of the amino acid sequence as shown in SEQID NO:1;

   said subunits of PP14 include one polypeptide having substantially all of the amino acid sequence as shown in SEQID NO:1; and

   said substance is effective in alleviating said immune system disorder.

11

2. A pharmaceutical preparation for treating an immune system disorder in a human comprising an active substance characterised in that said substance is a derivative of PP14, which, preferably, comprises a polypeptide including an amino acid sequence as shown in SEQID NO:1, or at least one sequential subset thereof, and/or an active mutein of PP14 comprising a mutant of a polypeptide including an amino acid sequence as shown in SEQID NO:1, or at least one sequential subset thereof; augmented by at least one additional molecule selected from the group consisting of glucose moieties, lipids, phosphate groups, acetyl groups, hydroxyl groups, saccharides, methyl groups, propyl groups, amino acids, and polymeric molecules.

3. A pharmaceutical preparation as claimed in claim 1 or claim 2, wherein at least one amino acid residue in the polypeptide has been modified by oxidation or reduction.

4. A pharmaceutical preparation as claimed in claim 1, wherein the PP14 comprises a dimer of two non-covalently linked protein subunits.

5. A pharmaceutical preparation as claimed in claim 4, wherein at least one of said subunits is employed as the active substance.

6. A pharmaceutical preparation as claimed in claim 5, wherein the subunit has an amino acid sequence as shown in SEQID NO:1.

7. A pharmaceutical preparation as claimed in claim 1, wherein the PP14 comprises two covalently linked protein subunits, each of which subunits has an amino acid sequence as shown in SEQID NO:1.

8. A pharmaceutical preparation as claimed in claim 1, wherein said fragment of PP14 has an amino acid sequence comprising residues 63 through 160 of SEQID NO:1.

9. A pharmaceutical preparation as claimed in claim 1, wherein said fragment has an amino acid sequence comprising residues 80 through 105 of SEQID NO:1.

10. A pharmaceutical preparation as claimed in claim 1, wherein said immune system disorder is selected from the group consisting of allergic conditions, autoimmune conditions, and inflammatory conditions.

11. A pharmaceutical preparation as claimed in claim 10, wherein said active substance is obtained from a source selected from the group consisting of mammalian placenta, mammalian blood, amniotic fluid, seminal plasma, cells in tissue culture, decidual cells, decidual organs, endometrial cells, endometrial organs, and sources containing eukaryotic or prokaryotic cells engineered to express PP14, muteins of Pp14, fragments of PP14 or subunits of PP14.

12. A pharmaceutical preparation as claimed in claim 10, wherein said immune system disorder is selected from the group consisting of arthritis, rheumatoid arthritis, asthma, graft-versus-host disease, organ rejection, systemic lupus erythematosis, atopic allergy, inflammatory tory bowel disease, multiple sclerosis, and allergic dermatitis.

13. A pharmaceutical preparation as claimed in claim 12, wherein said autoimmune conditons are manifested by infertility.

14. A pharmaceutical preparation as claimed in claim 12, wherein said substance is in admixture with a pharmaceutically acceptable carrier.

15. A pharmaceutical preparation as claimed in claim 1, wherein said immune system disorder comprises a lymphoproliferative disorder, preferably selected from the group consisting of malignant non-Hodgkin's lymphoma, Hodgkin's disease, and malignant histiocytosis.

16. A pharmaceutical preparation as claimed in claim 1, wherein said immune system disorder is a neoplastic disorder and, preferably leukemia.

17. A pharmaceutical preparation as claimed in claim 1, wherein said immune system disorder is a disorder resulting from the presence in said human of the virus which causes acquired immunodeficiency syndrome.

18. A pharmaceutical preparation for treating an immune system disorder in a human characterised by comprising monoclonal antibodies directed against an active substance as defined in either of claims 1 or 2, said monoclonal antibodies being effective in alleviating said immune system disorder.

19. A pharmaceutical preparation as claimed in claim 18, wherein said immune system disorder is a disorder resulting from the presence in said human of the virus which causes acquired immunodeficiency syndrome.

20. A pharmaceutical preparation as claimed in claim 18, wherein said monoclonal antibodies are in admixture with a pharmaceutically acceptable carrier.

21. A pharmaceutical preparation for inhibiting Interleukin-1 production in a human, characterised by comprising an active substance as defined in either of claims 1 and 2, said substance being effective to inhibit said Interleukin-1 production,

22. A pharmaceutical preparation as claimed in any of claims 10, 18 and 21 characterised by being in a form suitable for administration by means selected from the group consisting of intravenous injection, intramuscular injection, oral administration, topical administration, rectal administration, and inhalation.

23. A pharmaceutical preparation as claimed in claim 21, wherein said active substance is obtained from a source selected from the group consisting of mammalian placenta, mammalian blood, amniotic fluid, seminal plasma, cells in tissue culture, decidual cells, decidual organs, endometrial cells, endometrial organs, and sources containing eukaryotic cells or prokaryotic cells engineered to express PP14, muteins of PP14, fragments of PP14 or subunits of PP14.

24. A pharmaceutical preparation as claimed in claim 21, wherein said active substance is in admixture with a pharmaceutically acceptable carrier.

## SEQUENCE LISTING

```
       (1)   INFORMATION FOR SEQ ID NO:1:
 (i)         SEQUENCE CHARACTERISTICS:
       (A)   LENGTH:   819 base pairs
       (B)   TYPE:       nucleic acid sequence from which the
                         amino acid sequence was deduced.  N-
                         terminal sequence (about 20 amino acids)
                         also determined directly by amino acid
                         sequencing and the deduced sequence
                         agreed with this directly-determined
                         amino acid sequence at the N-terminus.
       (C)   STRANDEDNESS:   double stranded
       (D)   TOPOLOGY:   linear
 (x)         PUBLICATION INFORMATION:
       (A)   AUTHORS:  Julkunen, Mervi
                       Seppälä, Markku
                       Jänne, Olli A.
```

(B)  TITLE:  Complete Amino Acid Sequence of Human
             Placental Protein 14:  A Progesterone-
             Regulated Uterine Protein Homologous To
             β-Lactoglobulins
(C)  JOURNAL:  Proc. Natl. Acad. Sci. USA
(D)  VOLUME:  85
(F)  PAGES:  8845-8849
(G)  DATE:  DEC-1988
(K)  RELEVANT RESIDUES IN SEQ ID NO:1:  FROM 1 to 819


(xi)  SEQUENCE DESCRIPTION: SEQ ID NO:1:

```
CATCCCTCTG GCTCCAGAGC TCAGAGCCAC CCACAGCCGC AGCC                 44

ATG   CTG   TGC   CTC   CTG   CTC   ACC   CTG   GGC   GTG   GCC   77
Met   Leu   Cys   Leu   Leu   Leu   Thr   Leu   Gly   Val   Ala
-18                                           -10

CTG   GTC   TGT   GGT   GTC   CCG   GCC   ATG   GAC   ATC   CCC   110
Leu   Val   Cys   Gly   Val   Pro   Ala   Met   Asp   Ile   Pro
                                          1

CAG   ACC   AAG   CAG   GAC   CTG   GAG   CTC   CCA   AAG   TTG   143
Gln   Thr   Lys   Gln   Asp   Leu   Glu   Leu   Pro   Lys   Leu
                        10

GCA   GGG   ACC   TGG   CAC   TCC   ATG   GCC   ATG   GCG   ACC   176
Ala   Gly   Thr   Trp   His   Ser   Met   Ala   Met   Ala   Thr
                        20

AAC   AAC   ATC   TCC   CTC   ATG   GCG   ACA   CTG   AAG   GCC   209
Asn   Asn   Ile   Ser   Leu   Met   Ala   Thr   Leu   Lys   Ala
                  30

CCT   CTG   AGG   GTC   CAC   ATC   ACC   TCA   CTG   TTG   CCC   242
Pro   Leu   Arg   Val   His   Ile   Thr   Ser   Leu   Leu   Pro
            40

ACC   CCC   GAG   GAC   AAC   CTG   GAG   ATC   GTT   CTG   CAC   275
Thr   Pro   Glu   Asp   Asn   Leu   Glu   Ile   Val   Leu   His
      50

AGA   TGG   GAG   AAC   AAC   AGC   TGT   GTT   GAG   AAG   AAG   308
Arg   Trp   Glu   Asn   Asn   Ser   Cys   Val   Glu   Lys   Lys
60                                                        70

GTC   CTT   GGA   GAG   AAG   ACT   GGG   AAT   CCA   AAG   AAG   341
Val   Leu   Gly   Glu   Lys   Thr   Gly   Asn   Pro   Lys   Lys
                                          80

TTC   AAG   ATC   AAC   TAT   ACG   GTG   GCG   AAC   GAG   GCC   374
Phe   Lys   Ile   Asn   Tyr   Thr   Val   Ala   Asn   Glu   Ala
                                          90
```

| | | | | | | | | | | | |
|---|---|---|---|---|---|---|---|---|---|---|---|
| ACG | CTG | CTC | GAT | ACT | GAC | TAC | GAC | AAT | TTC | CTG | 407 |
| Thr | Leu | Leu | Asp | Thr | Asp | Tyr | Asp 100 | Asn | Phe | Leu | |
| TTT | CTC | TGC | CTA | CAG | GAC | ACC | ACC | ACC | CCC | ATC | 440 |
| Phe | Leu | Cys | Leu | Gln | Asp | Thr 110 | Thr | Thr | Pro | Ile | |
| CAG | AGC | ATG | ATG | TGC | CAG | TAC | CTG | GCC | AGA | GTC | 473 |
| Gln | Ser | Met | Met | Cys | Gln 120 | Tyr | Leu | Ala | Arg | Val | |
| CTG | GTG | GAG | GAC | GAT | GAG | ATC | ATG | CAG | GGA | TTC | 506 |
| Leu | Val | Glu | Asp | Asp 130 | Glu | Ile | Met | Gln | Gly | Phe | |
| ATC | AGG | GCT | TTC | AGG | CCC | CTG | CCC | AGG | CAC | CTA | 539 |
| Ile | Arg | Ala | Phe 140 | Arg | Pro | Leu | Pro | Arg | His | Leu | |
| TGG | TAC | TTG | CTG | GAC | TTG | AAA | CAG | ATG | GAA | GAG | 572 |
| Trp | Tyr | Leu | Leu 150 | Asp | Leu | Lys | Gln | Met | Glu | Glu | |
| CCG | TGC | CGT | TTC | TAG | CTCACCTCCG CCTCCAGGAA | | | | | | 607 |
| Pro | Cys 160 | Arg | Phe | AM 162 | | | | | | | |

GACCAGACTC CCACCCTTCC ACACCTCCAG AGCAGTGGGA CTTCCTCCTG 657

CCCTTTCAAA GAATAACCAC AGCTCAGAAG ACGATGACGT GGTCATCTGT 707

GTCGCCATCC CCTTCCTGCT GCACACCTGC ACCATTGCCA TGGGGAGGCT 757

GCTCCCTGGG GGCAGAGTCT CTGGCAGAGG TTATTAATAA ACCCTTGGAG 807

CATGAAAAAA AA 819